# EUROPEAN PATENT SPECIFICATION

(11) **EP 2 523 644 B1**
(45) Date of publication and mention of the grant of the patent: **09.12.2020**
(21) Application number: 11733459.9
(22) Date of filing: 14.01.2011
(51) Int. Cl.: A61J 1/20, A61J 1/05, B67D 7/00, F16K 41/12, C12M 1/26, F16K 41/10

(54) **FLUID TRANSFER DEVICE**
FLÜSSIGKEITSTRANSFERVORRICHTUNG
DISPOSITIF DE TRANSFERT DE FLUIDE

(30) Priority: 15.01.2010 US 688648; 15.01.2010 US 688654
(43) Date of publication of application: 21.11.2012
(73) Proprietor: Sartorius Stedim North America Inc., Bohemia, NY 11716 (US)
(72) Inventor: ZUMBRUM, Michael, A., New Oxford, PA 17350 (US)
(74) Representative: Vigand, Philippe
(86) International application number: PCT/US2011/021341
(87) International publication number: WO 2011/088350

(56) References cited:
- WO-A1-99/26580
- WO-A1-2004/044119
- WO-A1-2008/136720
- WO-A1-2009/122762
- WO-A2-2008/042285
- US-A- 4 018 059
- US-A- 4 537 593
- US-A- 4 861 239
- US-A1- 2005 016 620
- US-A1- 2006 020 283
- US-A1- 2006 197 049
- US-B1- 6 345 553
- US-B1- 6 515 677
- US-B1- 6 516 677

## Description

### TECHNICAL FIELD

This disclosure relates generally to fluid transfer devices and more specifically to a fluid transfer device for transferring fluid in a substantially aseptic manner.

### BACKGROUND

In the manufacturing and processing of many different products, it often is necessary to transfer fluid into or out of a closed processing system and do so in a substantially aseptic, hygienic, or sterile manner without breaching the closed nature of the system. In particular, the need to transfer fluid often arises in the manufacturing and processing of pharmaceuticals, biopharmaceuticals, or other biotechnology applications where processes are conducted in large process tanks, including but not limited to, the transfer of media solutions. The need for fluid transfer arises in other applications and industries as well, including but not limited to, the production of food, cosmetics, paint, chemicals, including hazardous chemicals, and the transfer and handling of semiconductor fluids.

Regardless of the industry, during transfers or sampling the fluid in tanks or other vessels must remain substantially free of contaminants. In addition, when making such transfers, it is desirable to keep the environment surrounding a vessel free from contamination by the contents of the vessel or a sample taken therefrom. It is often the case that throughout the manufacturing process there is a need to take multiple samples from the fluid or, in some circumstances, add additional fluid or media to the fluid in a vessel. To accomplish a substantially aseptic, hygienic, or sterile transfer, it is desirable to control the environment through which the fluid flows, for example, the pathway from a tank to a sample container should be substantially aseptic, hygienic, or sterile along the entire pathway. Furthermore, it is desirable that the fluid transfer device be safe for use, reliable, and of low-cost construction.

It is also desirable to transfer fluid using a device which is pre-sterilized and disposable. A pre-sterilized device avoids the need for an operator to prepare the device for use. In addition, a disposable device avoids the time consuming and laborious task of sterilizing sampling equipment. Further, such sterilization can damage the fluid transfer device and render it useless before its first use.

Pre-sterilized sampling devices that allow for substantially aseptic transfers are disclosed in pending PCT Application WO 2010/008395 (PCT/US2008/070482), owned by the assignee of the present invention. Other similar devices are known from US6516677 B1 and US2006197049 A1.

In some instances there arises the need to transfer a media solution having a high particulate content, that contains particles, or is of high viscosity. These media solutions may clog a cannula or hypodermic needle found in some fluid transfer devices. Devices that utilize a hypodermic needle or cannula, like that disclosed in PCT Application No. PCT/US2008/070482, may clog if particles or a media solution with a high particulate concentration or viscosity is transferred through the device. Such media solutions necessitate larger openings to accommodate particulates or increased viscosity without clogging the transfer device. One sampling device that is capable of transferring fluids with particulates is disclosed in U.S. Patent No. 7,293,477. However, that device lacks a substantially aseptic, hygienic or sterile cavity in the fluid transfer device that ensures that the fluid in the vessel from which a sample may be drawn remains substantially free of contaminants while, at the same time, ensuring that the environment surrounding a vessel remains free from contamination by the contents of the sample.

In addition, there arises the need to transfer media solutions containing cells, live cultures, or proteins. Such media solutions require low sheer and other physical forces to avoid damage to the media's content during a transfer. Devices using a cannula or hypodermic needle may cause such damage.

In view of the above, there exists a need for a fluid transfer device that is inexpensive, pre-sterilized, disposable, capable of being used with standard industrial ports commonly found in fluid receptacles, and capable of use in common industrial settings, such as those found in the pharmaceutical, biopharmaceutical, or other high purity industries, where there is often the need to transfer fluids with particles or high particulate concentrations.

### SUMMARY

U.S. Application US 2010/0123094 (12/688,648) and US 2010/0133459 (12/688,654) filed on January 15, 2010, to which priority is claimed above. The subject-matter of the present invention is defined by the features of independent claim 1. Further preferred embodiments of the present invention are defined in the dependent claims.

Briefly described, a fluid transfer device is disclosed for transferring fluid into or from a fluid receptacle, such as a tank, in a substantially aseptic manner. As used herein, the term "aseptic" includes aseptic, hygienic, or sterile conditions. In the following, reference is made to a plurality of examples which are useful for the understanding of the present invention. In a first
preferred and illustrated example, the device is configured to sample a fluid or other media, such as slurry, from a fluid vessel. As used herein, "fluid" includes high particulate fluids, slurries, or any high viscosity fluid, including without limitation soy-based media or media containing anti-foaming agents, such as silica-based agents. It should be understood, however, the fluid transfer devices disclosed herein are not limited to taking samples from a process vessel, and may be used for any type of fluid or media transfer into or out of a fluid vessel, especially when there is a need to maintain aseptic conditions both within the fluid pathway and to protect the environment from the transferred fluid. Additionally, the fluid transfer device disclosed herein provides a fluid pathway that minimizes sheer and other forces that may damage cells or proteins in a media solution.

The device comprises a body with an elongate passage extending through the body. The body has a proximal end and a distal end. The proximal end is that end closest to the fluid vessel into or from which fluid is transferred. The distal end is that end furthest away from the fluid vessel into or from which fluid is transferred. A longitudinally displaceable substantially hollow shaft is disposed in and extends along the passage in the body. The hollow interior of the shaft provides part of the fluid pathway created upon actuation of the device. The shaft has a proximal end and a distal end that corresponds with the proximal and distal ends of the passage. When actuated, the shaft moves longitudinally within the passage and moves between a first and second position. In its first position, the shaft is displaced toward the distal end of the passage. In its second position the shaft is displaced toward the proximal end of the passage.

A plug is attached to the proximal end of the shaft and, when the shaft is at its first position, the plug seals the passage at the proximal opening. A diaphragm seals the passage at a location intermediate the plug and the distal end of the passage. The shaft extends through and is sealingly secured to the diaphragm. The shaft has a fluid transfer opening located between the plug and the diaphragm to provide a pathway for fluid transfer when the device is activated. Longitudinal displacement of the shaft towards its second position moves the plug and opens the passage. The diaphragm attached to the shaft stretches to accommodate the movement of shaft while maintaining its seal about the shaft and thus maintaining a seal of the distal end of the passage. When the shaft moves the plug and the passage opens, a fluid flow path is established through the fluid transfer opening in the shaft and through its substantially hollow interior. The passage between the plug and diaphragm may be substantially aseptic. In addition, the entire pathway that fluid flows from the fluid vessel to a sample container may be substantially aseptic. The plug may be partially disposed inside the proximal end of the passage before displacement of the shaft from its first position.

The device also includes a tank mount that allows the device to be connected to any standard industrial vessel port or, if desired, a customized port. The tank mount is attached to the fluid transfer device and is also substantially aseptic to meet the needs of the end user. The tank mount contains an opening through which the shaft may pass when the shaft is longitudinally displaced in the proximal direction. The tank mount may include a groove located in the tank mount and a seal located in the groove that allows the formation of a seal between a tank mount and a tank. The tank mount may also comprise a threaded stud for mounting the fluid transfer device to a tank.

In a second example not covered by the present invention, the longitudinally displaceable substantially hollow shaft has a first position displaced toward the proximal end of the passage and a second position displaced toward the distal end of the passage. The shaft is axially biased toward the proximal end of the passage in its first position. Longitudinal displacement of the shaft towards its second position moves the plug and opens the passage. The diaphragm attached to the shaft stretches to accommodate the movement of shaft while maintaining its seal about the shaft and thus maintains a seal of the distal end of the passage. When the shaft moves the plug and the passage opens, a fluid flow path is established between the open end of the passage and a fluid transfer opening in the shaft and through its substantially hollow interior. Like with the first preferred and illustrated example, the passage between the plug and diaphragm may be substantially aseptic and the entire pathway that fluid flows to or from the fluid vessel may also be substantially aseptic. Also like with the earlier example, the plug may be partially disposed inside the proximal end of the passage before displacement of the shaft from its first position.

A third alternative example not covered by the present invention, the device comprises a body with an elongate passage extending through the body. The body has a proximal end and a distal end. The proximal end is that end closest to the fluid vessel into or from which fluid is transferred. The distal end is that end furthest away from the fluid vessel into or from which fluid is transferred. A longitudinally displaceable shaft is disposed in and extends along the passage in the body. The shaft has a proximal end and a distal end that corresponds with the proximal and distal ends of the passage. When actuated, the shaft moves longitudinally within the passage and moves between a first and second position. In its first position, the shaft is displaced toward the distal end of the passage. In its second position the shaft is displaced toward the proximal end of the passage.

A plug is attached to the proximal end of the shaft and, when the shaft is at its first position, the plug seals the passage at the proximal opening. A diaphragm seals the passage at a location intermediate the plug and the distal end of the passage. The shaft extends through and is sealingly secured to the diaphragm. A fluid transfer opening or side port is located in the passage between the plug and diaphragm. The fluid transfer opening provides a pathway for fluid transfer when the device is actuated. A conduit is connected to the fluid transfer opening to assist with the transfer of fluid and to which further conduit or flexible tubing may be attached. Longitudinal displacement of the shaft towards its second position moves the plug and opens the passage. The diaphragm attached to the shaft stretches to accommodate the movement of shaft while maintaining its seal about the shaft and thus maintaining a seal of the distal end of the passage. When the shaft moves the plug and the passage opens, a fluid flow path is established through the passage and fluid transfer opening and further through the conduit attached to the fluid transfer opening. The passage between the plug and diaphragm may be substantially aseptic. In addition, the entire pathway that fluid flows from the fluid vessel to a sample container may be substantially aseptic. The plug may be partially disposed inside the proximal end of the passage before displacement of the shaft from its first position.

The third alternative example of the device also includes a tank mount that allows the device to be connected to any standard industrial vessel port or, if desired, a customized port. The tank mount is attached to the fluid transfer device and is also substantially aseptic to meet the needs of the end user. The tank mount contains an opening through which the shaft may pass when the shaft is longitudinally displaced in the proximal direction. The tank mount may include a groove located in the tank mount and a seal located in the groove that allows the formation of a seal between a tank mount and a tank. The tank mount may also comprise a threaded stud for mounting the fluid transfer device to a tank.

In a fourth example not covered by the present invention, the longitudinally displaceable shaft has a first position displaced toward the proximal end of the passage and a second position displaced toward the distal end of the passage. The shaft is axially biased toward the proximal end of the passage in its first position. Longitudinal displacement of the shaft towards its second position moves the plug and opens the passage. The diaphragm attached to the shaft stretches to accommodate the movement of shaft while maintaining its seal about the shaft and thus maintains a seal of the distal end of the passage. When the shaft moves the plug and the passage opens, a fluid flow path is established between the open end of the passage and a fluid transfer opening in the passage and out through conduit attached to the fluid transfer opening. Like with the alternative preferred and illustrated example, the passage between the plug and diaphragm may be substantially aseptic and the entire pathway that fluid flows to or from the fluid vessel may also be substantially aseptic. Also like with the earlier alternative example, the plug may be partially disposed inside the proximal end of the passage before displacement of the shaft from its first position.

The fluid transfer devices disclosed herein preferably comprise a tab assembly to which the shaft is connected. The tab assembly controls the displacement of the shaft through the passage regardless of whether displacement occurs in the proximal direction, that is, pushing the tab, or in the distal direction, that is, pulling the tab. The tab assembly preferably comprises a tab guide. Longitudinal displacement of the tab assembly displaces the tab guide through a portion of the passage. Preferably, the shaft is axially biased longitudinally (either distally or proximally) to maintain the plug in a position that seals the proximal end of the passage. In this example, a bias may retract the shaft to a first position after displacement.

The devices may also comprise a retaining cap at the distal end of the body. The retaining cap may comprise an opening through which the shaft and tab guide pass. The retaining cap engages the distal end of the body and further comprises restraining means allowing the tab guide and the shaft to move through the retaining cap and stop at a predetermined position. The restraining means may comprise an axial channel extending along the tab guide and a detent extending from the opening in the retaining cap. The detent extends from the opening in the retaining cap into the axial channel along the tab guide. The detent limits the longitudinal displacement of the tab guide in the longitudinal direction and thus assists with keeping the tab guide and entire tab assembly from coming out of the retaining cap and away from the body of the device during operation.

A single fluid transfer device is preferably joined together with additional fluid transfer devices as described in herein to form an assembly having a plurality of bodies. Using multiple devices assembled together allows a user to take multiple samples from a single fluid vessel, either simultaneously or, as is more often the case, over the course of a process. Each sample can be taken without exposing the fluid vessel to the environment and without exposing the environment to the transferred fluid. Alternatively, an assembly of devices may be used to make multiple transfers of media or fluid into a fluid vessel.

Also provided is a kit for transferring fluids or media comprising a fluid transfer device as described herein. The fluid transfer device may comprise one or more bodies joined together. The kit may also contain a tank mount, one or more lengths of flexible tubing, and a plurality of sample containers. In an example, the kit is rendered substantially aseptic and packaged to maintain a substantially aseptic state before use.

Thus, unique fluid transfer devices with a substantially aseptic fluid pathway are disclosed that possess distinct attributes and represents distinct improvements over the prior art. These and other aspects, features, and advantages of the fluid transfer devices of this disclosure will be better understood and appreciated upon review of the detailed description set forth below when taken in conjunction with the accompanying drawing figures, described briefly below. According to common practice, the various features of the drawings may not be drawn to scale. Dimensions and relative sizes of various features and elements in the drawings may be shown enlarged or reduced to illustrate more clearly the embodiments of the invention.

### BRIEF DESCRIPTION OF THE DRAWINGS

Fig. 1 depicts a fluid sampling device assembly and flexible tubing.
Fig. 2 is a cross-section of a fluid transfer device assembly with the shaft in the first position.
Fig. 3 is a cross section of a fluid transfer device with the shaft in the first position.
Fig. 4 is a perspective view of a fluid sampling device assembly with flexible tubing and a sample container attached with the shafts in the first position.
Fig. 5 is a cross-section of a fluid transfer device with the shaft in the second position.
Fig. 6 is a perspective view of an embodiment of a stretched diaphragm.
Fig. 7 is a perspective view of an embodiment of a tab assembly.
Fig. 8 is a perspective view of an embodiment of a retaining cap.
Fig. 9 is a cross-section of a fluid transfer device with the shaft in the first position.
Fig. 10 is a cross-section of a fluid transfer device with the shaft in the second position.
Fig. 11 depicts a fluid sampling device assembly and flexible tubing.
Fig. 12 is a cross-section of a fluid transfer device assembly with the shaft in the first position.
Fig. 13 is a cross section of a fluid transfer device with the shaft in the first position.
Fig. 14 is a perspective view of a fluid sampling device assembly with flexible tubing and a sample container attached with the shafts in the first position.
Fig. 15 is a cross-section of a fluid transfer device with the shaft in the second position.
Fig. 16 is a perspective view of an embodiment of a stretched diaphragm.
Fig. 17 is a perspective view of an embodiment of a tab assembly.
Fig. 18 is a perspective view of an embodiment of a retaining cap.
Fig. 19 is a cross-section of a fluid transfer device with the shaft in the first position.
Fig. 20 is a cross-section of a fluid transfer device with the shaft in the second position.

### DETAILED DESCRIPTION

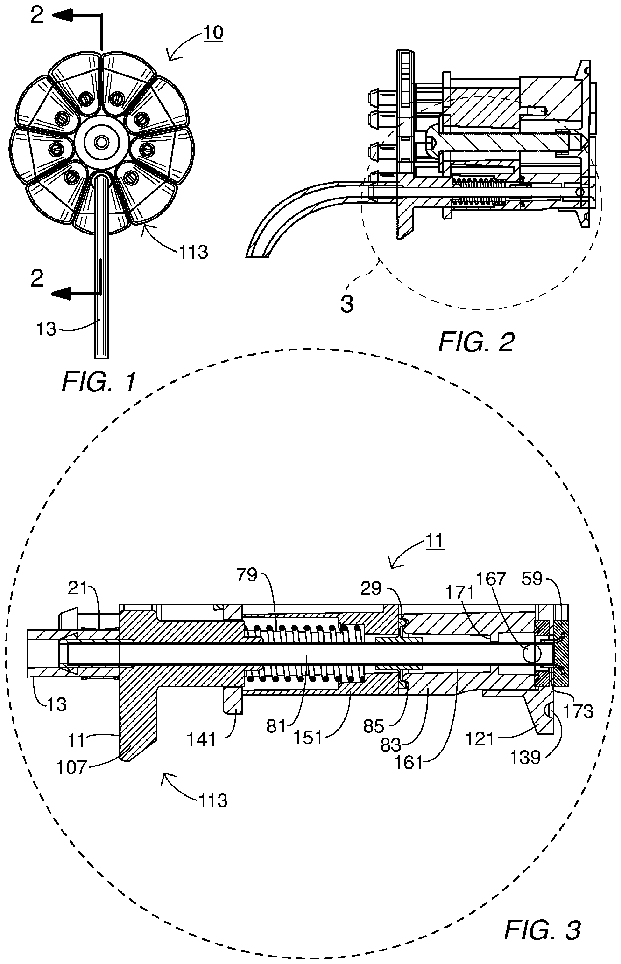

Referring now in more detail to the drawing figures, wherein like reference numerals indicate like parts throughout the several views, Fig. 1 depicts a first exemplary fluid sampling device assembly, in this case a device suitable for use with fluids having particulates, such as slurry, a liquid having a high percent solids, or a high viscosity liquid, and flexible tubing according to the present disclosure. It should be understood that the fluid transfer device is not limited to use with fluids with particulates or having a high viscosity and may be used with a variety of fluids, including those having no or very little particulate content or low viscosity.

The fluid transfer device illustrated in Fig. 1 depicts nine fluid transfer devices joined together as an assembly of multiple fluid transfer devices that allows for multiple samples to be drawn from a single fluid vessel or, alternatively, for fluid or media to be added to a fluid vessel, such as with inoculations, without opening the fluid vessel to the surrounding environment. The fluid transfer device assembly is not limited to this number of joined fluid transfer devices, but may incorporate more or fewer fluid transfer devices in an assembly. In general, the exemplary fluid transfer device assembly **10** has a circular outer configuration defined by body sections discussed in more detail below. The tab assemblies **113** shown in exemplary fluid transfer device assembly **10** are moved to longitudinally displace shafts from their first position to their second position. As shown in Fig. 1, the shafts are in their first positions.

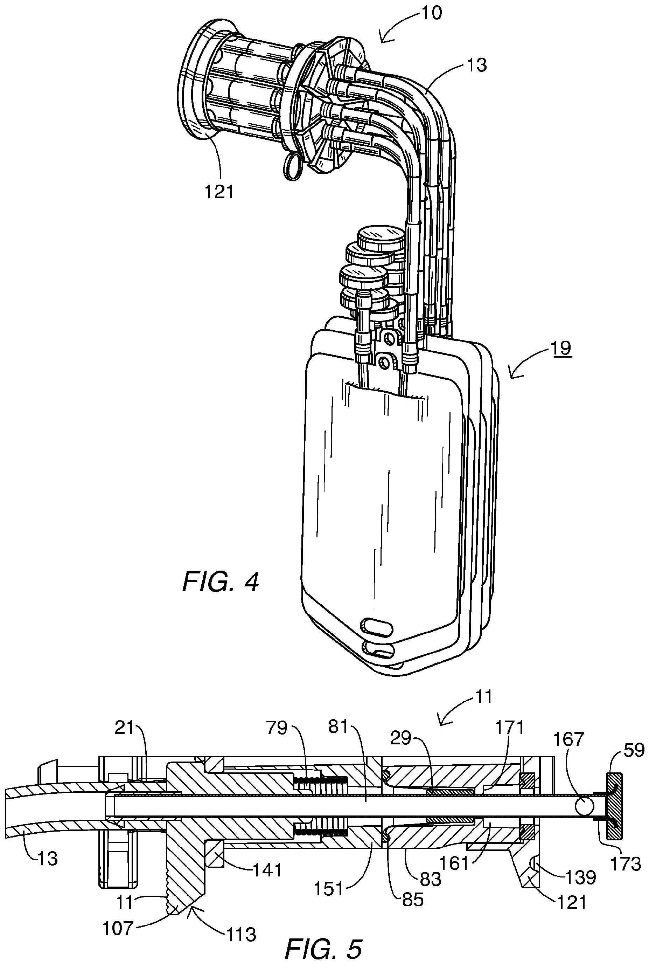

In the fluid transfer device assembly illustrated in Fig. 1, flexible tubing **13** is attached to a single fluid transfer device. For clarity, only a portion of the flexible tubing attached is shown. Flexible tubing may be attached to any number of fluid transfer devices of the assembly depending on the number of transfers needed. Each piece of flexible tubing **13** may be directed away from the fluid transfer device assembly and to a fluid sample container **19** as shown in Fig. 4 and described in greater detail below. The device may be used, for example, to take multiple fluid samples from a fluid vessel, such as a tank, wherein a process is running. The samples may be taken simultaneously or over differing time intervals. The fluid transfer device assembly **10** may be connected to a fluid vessel, such as a tank, prior to a process being performed in the tank. The flexible tubing **13** may be connected to the fluid transfer device assembly **10** prior to connecting to the fluid transfer device **11** to the tank. Likewise, the sample containers **19** may be connected to the flexible tubing **13** prior to connecting the flexible tubing **13** to the fluid transfer device assembly **10** and before the fluid transfer device assembly **10** is connected to a fluid vessel. However, it is understood that the fluid transfer device assembly **10**, the flexible tubing **13**, and the sample containers **19** may be attached in any order necessary to use the device to transfer fluid. Sample fluid containers suitable for use with the fluid transfer device include without limitation, bags, bottles, syringes, other tanks, tubing, manifolds, vials, or any combination thereof. If desired, more rigid tubing or conduit may be used in place of flexible tubing **13** in which case venting may be necessary.

The flexible tubing **13** may be connected to the fluid transfer device assembly **10** by a crimped collar **21** that affixes the tubing to the distal end of the shaft. However, the flexible tubing **13** may be connected by other means, such as by press-fit, an adhesive, or the like. Likewise, the flexible tubing **13** can be connected to the sample container in similar means.

Fig. 2 shows a cross-section of the fluid transfer device assembly **10.** Fig. 3 shows a detailed cross-section of an exemplary fluid transfer device found in the assembly shown in Figs. 1 and 2. The fluid transfer device **11** comprises an elongate passage therethrough, flexible tubing **13**, a crimped collar **21**, a plug **59**, a spring **79**, a longitudinally displaceable substantially hollow shaft **81**, a lower portion of a body **83**, a diaphragm seat **85**, a tab assembly **113**, ridges **111** on a tab **107**, a tank mount **121**, a groove in the tank mount **139**, a retaining cap **141**, an upper portion of a body **151**, a substantially aseptic chamber **161**, a fluid transfer opening **167** in the shaft, a shaft alignment aperture **171**, and plug supports **173.** With respect to the fluid transfer device **11** and the components therein, there is a proximal end and a distal end. The proximal end is that end closest to the fluid vessel into or from which fluid is transferred. In Fig. 3, the proximal end of the device is the end of the device where the plug **59** is found. The distal end of the device is that end furthest away from the fluid vessel into or from which fluid is transferred and in Fig. 3, the distal end is the end where the flexible tubing **13** is attached to the device. As used throughout this disclosure, the terms "proximal" and "distal" refer to these ends of the fluid transfer devices disclosed herein. However, it should be understood that if a component is located in a proximal or distal direction, it does not need to be at the complete proximal or distal or end of the device. Rather, the component may merely be located in that general direction from the point of reference. Thus the terms "proximal" and "distal" are relative and should be understood to merely aid reference in this disclosure. Similarly, it should be understood that reference to transferring fluid from a vessel is but one intended use of the devices disclosed herein and the devices may be and are intended to be used to transfer fluid or media into a fluid vessel.

With continued reference to Fig. 3, the longitudinally displaceable substantially hollow shaft **81** (referred to herein occasionally as just "shaft") has a proximal and distal end that corresponds with the proximal and distal ends of the passage. In operation, the shaft travels between a first position and a second position. As shown in Fig. 3, the shaft is in its first position. The shaft extends through and is sealingly attached to a diaphragm **29.** A plug **59** is attached to the proximal end of the shaft. The plug **59** is secured to the shaft using supports **173.** The plug may be constructed from silicone or, preferably, from platinum-cured silicone. The plug may also be constructed from a solvent resistant fluoroelastomer, such as a perfluoropolyether elastomer. However, it is understood that plug **59** may be constructed of any suitable material. In Fig. 3, the plug **59** is located outside the proximal end of the passage prior to displacement of the shaft from its first position. However, the plug **59** may also be located at least partially within the inside the passage prior to displacement of the shaft from its first position. The plug may be shaped and located to effectively seal the proximal end of the passage when the shaft is in its first position.

The shaft is attached to and extends through tab assembly **113** on the distal end of the device. Flexible tubing **13** may be securely attached to the shaft using, for example, a crimped collar **21.** The shaft extends through and is sealingly attached to a diaphragm **29.** The shaft **81** is substantially hollow to allow a pathway for fluids to travel and a fluid transfer opening **167** is located in the shaft between the plug **59** and the diaphragm **29.** The substantially hollow nature of the shaft allows for fluid to move into the fluid transfer opening **167** and through the length of the shaft to the flexible tubing **13** and then on to sample containers **19** (not shown in Fig. 3). Alternatively, when fluid or media is transferred into a fluid vessel, the fluid pathway is essentially reversed. The space along the passage between the plug **59** and diaphragm **29** creates a chamber **161.** This chamber remains substantially aseptic throughout operation and acts to keep the fluid transfer within a closed system throughout an entire fluid transfer process.

With continued reference to Fig. 3, the shaft is disposed within a body that is constructed from an upper body portion **151** and a lower body portion **83** that, when joined together, form a unitary body. It should be understood that the body may be formed as a single structure or from two or more portions. The purpose of the body is to enclose and create a passageway in which the components of the fluid transfer device are located and thus the construction of the body may vary to accommodate the number of fluid transfer devices joined in an assembly. As shown, the body has a substantially cylindrical outer portion. Such shape provides for a compact organization of fluid transfer devices, is easily handled, and is similar in shape to the tank mount. Preferably, the body is constructed from high temperature glass reinforced polyester. Alternatively, the body may be constructed of polyvinylidene fluoride, polycarbonate (which provides high temperature resistance), polyether ether ketone (PEEK), or Ultem® (polyetherimide). However, it should be understood that the body may be constructed of any suitable material and its construction is not limited to the materials listed herein. Preferably the body, and in particular the lower body portion **83**, contains an alignment aperture **171** that guides the shaft while moving between its first and second positions.

Also depicted in Fig. 3 is tank mount **121.** The fluid sample device **11** or fluid sample device assembly **10**, as shown in Fig. 1, is joined to a tank mount that allows the fluid sample device or assembly to be joined to any number of fluid vessels that are used in industrial settings. The tank mount is preferably constructed of 316L stainless steel. However, it should be understood that the tank mount may be constructed of any suitable material. The tank mount may also include a groove **139** in which a seal is located to assist with a substantially aseptic connection of the fluid transfer device to a fluid vessel.

In operation the shaft **81** moves between two positions. In its first position, the shaft is displaced toward the distal end of the passage, as shown in Fig. 3. The shaft **81** may be biased in a first position that displaces the shaft toward the distal end of the passage. In other words, the shaft **81** is forced in the distal direction and the plug **59** seals the proximal end of the passage. The bias on the shaft **81** may be created by, for example, the placement of a spring **79**, such as shown in Fig. 3. While the shaft is in this first position the plug **59** seals the passage through the body of the device at its proximal end. The diaphragm **29** seals the passage at a location distal from the plug **59** and thus creates the substantially aseptic chamber **161.**

In its second position, as shown in Fig. 5, the shaft is displaced in a proximal direction toward the proximal end of the passage, for example, by a user depressing the tab and pushing the shaft proximally into a fluid vessel. Once displaced, the fluid transfer opening is extended into the fluid of the fluid vessel and a fluid pathway is created through the fluid transfer opening and through the shaft's substantially hollow interior. The fluid then travels through the shaft, through flexible tubing and into a fluid sample container. The shaft is maintained in this second position until a sufficient sample of fluid is collected. Alternatively, fluid or media may be added to a fluid vessel by forcing the fluid or media to be added along this fluid pathway and into the fluid vessel.

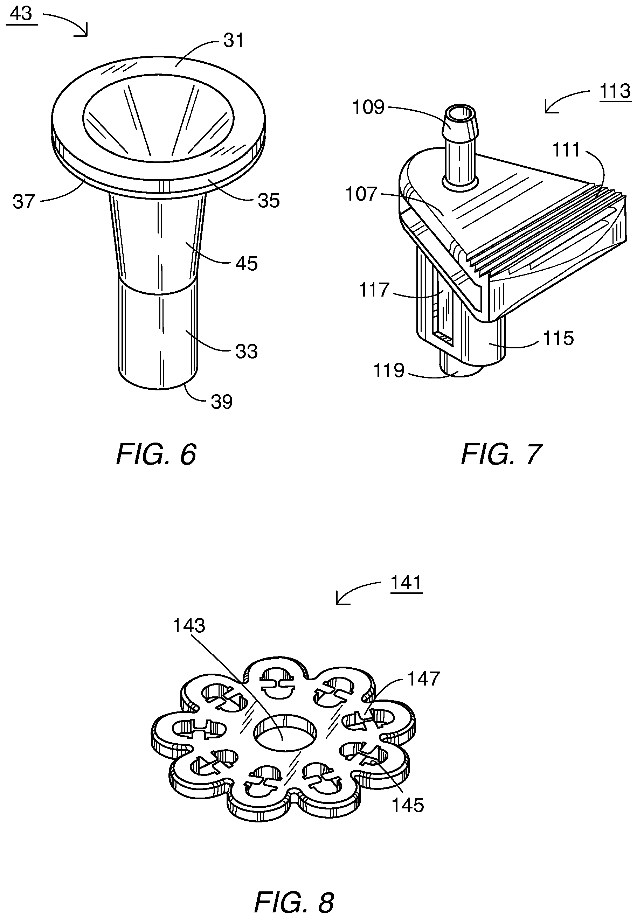

When the shaft is in its second position the diaphragm **29** stretches while maintaining a seal around the shaft. Fig. 6 illustrates the diaphragm as it appears when stretched along its axis. The stretched diaphragm **43** is made of flexible material and is capable of stretching in at least the direction of its axis. In this illustration, the stretched diaphragm **43** has a top face **31**, a central body **33**, a side **35**, an annular bead **37**, and a central opening **39.** The central opening **39** is substantially cylindrical to receive the shaft **81.** The body receives the annular bead **37** in a seat **85** as shown in Fig. 3. As used in the fluid transfer device disclosed herein, a shaft (see Figs. 3, 5, 9 and 10) extends through the central opening **39** and is secured to the diaphragm **29** by molding, for example, the diaphragm **29** around the shaft when the diaphragm is produced. However, the diaphragm **29** may be secured to a shaft through other means such as with adhesives or sealants. It is further understood that the central opening **39** may have various shapes.

As shown in Fig. 7, a tab assembly **113** comprises a tab **107**, a barb **109** (to which the shaft **81** is fluidly connected from beneath and to which flexible tubing **13** may be connected from above), ridges **111** to facilitate operation, a tab guide **115** to guide the tab during use, and an axial channel **117** in the tab guide **115.** The shaft **81** may be connected to the tab at the opening **119.** Furthermore, a spring, such as that depicted in Fig. 3, may be fitted around the extension in which opening **119** is located. Such arrangement is depicted in detail in Fig. 3.

Fig. 8 illustrates a retaining cap **141**, a central opening **143**, and openings **145** through which tab guides, such as those depicted in Fig. 7, pass, and detents **147.** In an assembled fluid transfer device, the retaining cap **141** is located at the distal end of the upper portion of the body as shown in Fig. 3. The retaining cap **141** may be constructed of a flexible material, such as polycarbonate or polyolefin. Preferably, the retaining cap is constructed of polycarbonate. However, the retaining cap may be constructed from any suitable material. The detents **147** travel in the axial channel **117** of the tab guide **115.** Further, the bottom surface of tab **107**, that is, the side opposite the ridges **111**, contacts the top of the retaining cap **141**, thereby limiting the axial travel of tab assembly **113.** After a sample is taken, and when the tab **107** is retracted in a distal direction, the extended detents **147** keep the tab assembly **113** from coming out of the retaining cap **141** and potentially tearing the diaphragm and breaking the substantially aseptic state of the fluid transfer device and, in particular, the substantially aseptic chamber **161.**

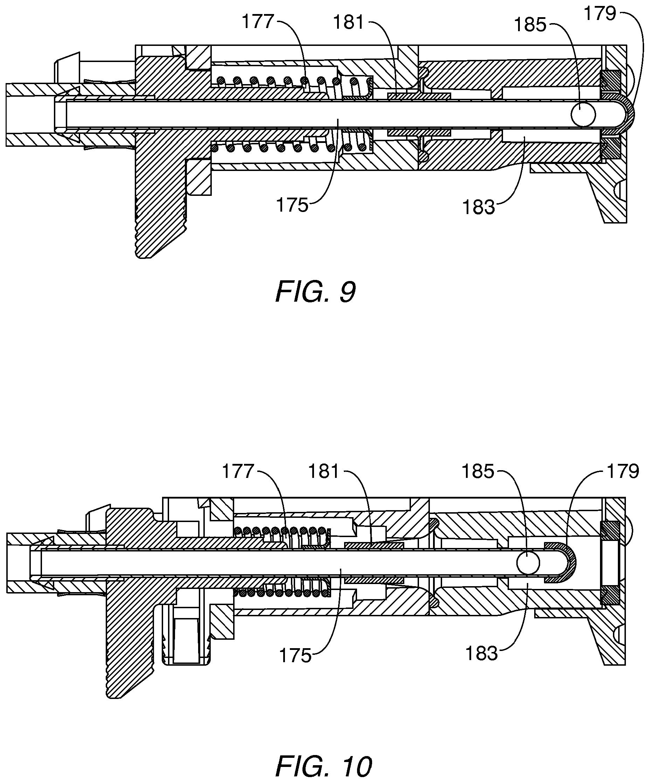

In a second example of a fluid transfer device not covered by the present invention, and as disclosed in Fig. 9, a longitudinally displaceable substantially hollow shaft **175** is disposed in and extend along a passage extending through the body of a fluid transfer device. The shaft **175** has a closed proximal end and a substantially hollow interior. The shaft **175** is open on the distal end for fluid communication with the flexible tubing or other conduit. The shaft **175** moves between two positions wherein in its first position the shaft is displaced toward the proximal end of the passage. The shaft **175** may be biased in a first position that displaces the shaft toward the proximal end of the passage. The bias may be created, for example, by the placement of a spring **177**, such as shown in Fig. 9. While the shaft is in this first position a plug **179** seals the passage through the body of the device at its proximal end. Plug **179** is attached to the closed proximal end of the shaft **175.** A diaphragm **181** seals the passage at a location distal from the plug. The portion of the passage between the plug **179** and diaphragm **181** is a chamber **183** that is substantially aseptic.

In its second position, as shown in Fig. 10, the shaft is displaced toward the distal end of the passage, for example, by the user pulling the tab and thus pulling the shaft **175** and plug **179** distally away from a fluid vessel. Once displaced, the shaft **175** and plug **179** no longer create a seal at the proximal end of the passage and fluid now enters the passage up to the location of the diaphragm **181** which maintains a seal around the distally displaced shaft. A fluid flow path is now created between the open end of the passage and the fluid transfer opening **185** and through the shaft's substantially hollow interior. The fluid then travels distally through the flexible tubing and into a fluid sample container. The shaft is left in this second position until a sufficient sample of fluid is collected. Alternatively, the device in this example may be used to transfer fluid or media into a fluid vessel as discussed in greater detail above. When the shaft is in its second position the diaphragm 181 stretches while maintaining a seal around the shaft. Fig. 6 illustrates the diaphragm as it appears when stretched along its axis.

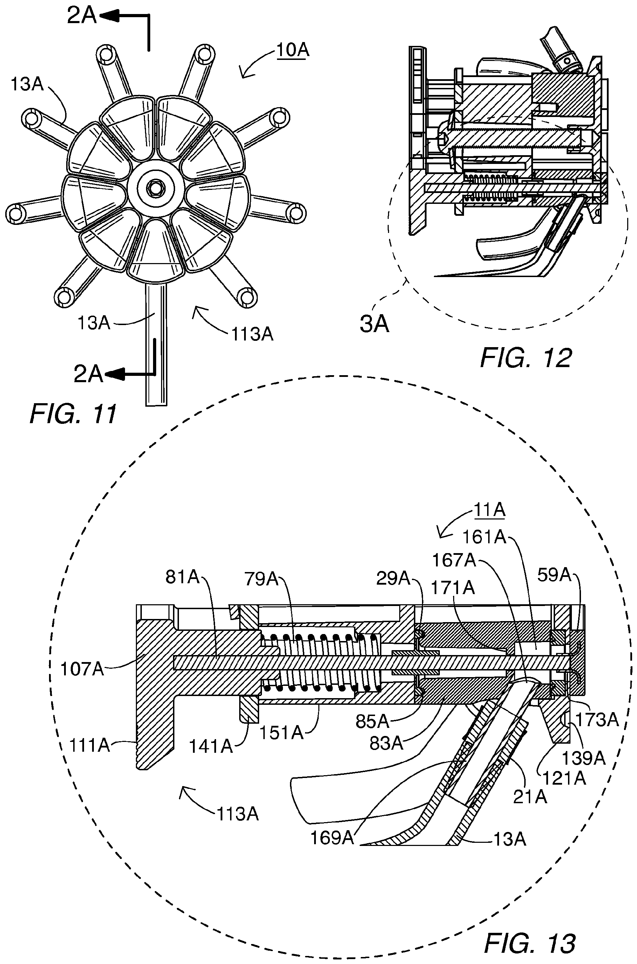

Referring now to a third alternative example not covered by the present invention, Fig. 11 depicts an alternative exemplary fluid sampling device assembly, in this case a device suitable for use with fluids having particulates, such as slurry, a liquid having a high percent solids or a high viscosity liquid, and flexible tubing according to the present disclosure. It should be understood that the fluid transfer device is not limited to use with fluids with particulates or having a high viscosity and may be used with a variety of fluids, including those having no or very little particulate content or low viscosity.

The fluid transfer device illustrated in Fig. 11 depicts nine fluid transfer devices joined together as an assembly of multiple fluid transfer devices that allows for multiple samples to be drawn from a single fluid vessel or, alternatively, for fluid or media to be added to a fluid vessel, such as with inoculations, without opening the fluid vessel to the surrounding environment. The fluid transfer device assembly is not limited to this number of joined fluid transfer devices, but may incorporate more or fewer fluid transfer devices in an assembly. In general, the exemplary fluid transfer device assembly **10A** has a circular outer configuration defined by body sections discussed in more detail below. The tab assemblies **113A** shown in exemplary fluid transfer device assembly **10A** are moved to longitudinally displace shafts from their first position to their second position. As shown in Fig. 11, the shafts are in their first positions.

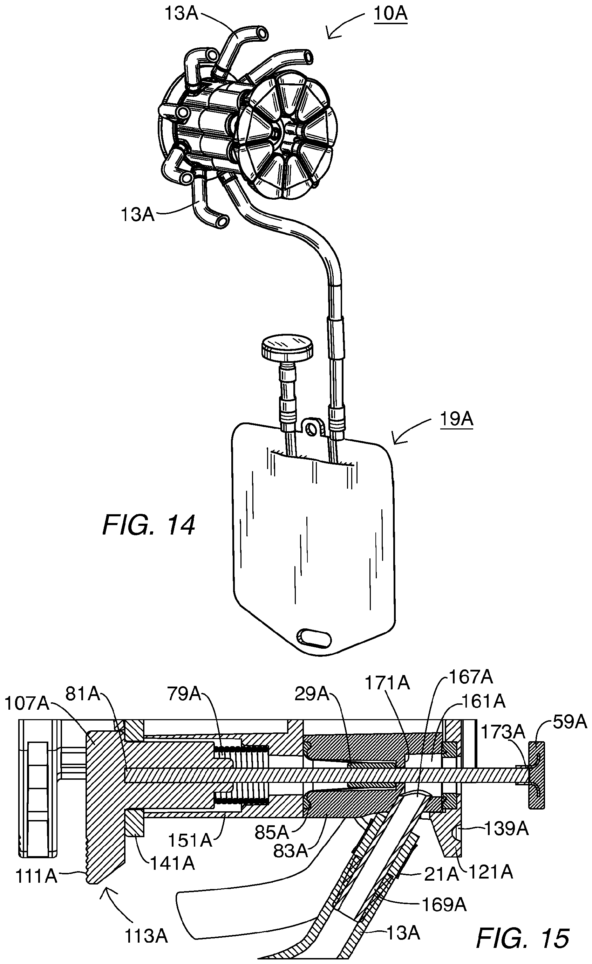

In the fluid transfer device assembly illustrated in Fig. 11, flexible tubing **13A** is attached to a multiple fluid transfer devices. For clarity, only a portion of the flexible tubing attached to each is shown. In use, flexible tubing may be attached to any number of fluid transfer devices of the assembly depending on the number of transfers needed. Each piece of flexible tubing **13A** may be directed away from the fluid transfer device assembly and to a fluid sample container **19A** as shown in Fig. 14 and described in greater detail below. The device may be used, for example, to take multiple fluid samples from a fluid vessel, such as a tank, wherein a process is running. The samples may be taken simultaneously or over differing time intervals. The fluid transfer device assembly **10A** may be connected to a fluid vessel, such as a tank, prior to a process being performed in the tank. The flexible tubing **13A** may be connected to the fluid transfer device assembly **10A** prior to connecting to the fluid transfer device **11A** to the tank. Likewise, the sample containers **19A** may be connected to the flexible tubing **13A** prior to connecting the flexible tubing **13A** to the fluid transfer device assembly **10A** and before the fluid transfer device assembly **10A** is connected to a fluid vessel. However, it is understood that the fluid transfer device assembly **10A**, the flexible tubing **13A**, and the sample containers **19A** may be attached in any order necessary to use the device to transfer fluid. Sample fluid containers suitable for use with the fluid transfer device include without limitation, bags, bottles, syringes, other tanks, tubing, manifolds, vials, or any combination thereof. If desired, more rigid tubing or conduit may be used in place of flexible tubing **13A** in which case venting may be necessary.

The flexible tubing **13A** may be connected to the fluid transfer device assembly **10A** by a crimped collar **21A** that affixes the tubing to the distal end of the shaft. However, the flexible tubing **13A** may be connected by other means, such as by press-fit, an adhesive, or the like. Likewise, the flexible tubing **13A** can be connected to the sample container in similar means.

Fig. 12 shows a cross-section of the fluid transfer device assembly **10A.** Fig. 13 shows a detailed cross-section of an exemplary fluid transfer device found in the assembly shown in Figs. 11 and 12. The fluid transfer device **11A** comprises an elongate passage therethrough, flexible tubing **13A**, a crimped collar **21A**, a plug **59A**, a spring **79A**, a longitudinally displaceable shaft **81A**, a lower portion of a body **83A**, a diaphragm seat **85A**, a tab assembly **113A**, ridges **111A** on a tab **107A**, a tank mount **121A**, a groove in the tank mount **139A**, a retaining cap **141A**, an upper portion of a body **151A**, a substantially aseptic chamber **161A**, a fluid transfer opening **167A**, a fluid transfer conduit **169A**, a shaft alignment aperture **171A**, and plug supports **173A.** With respect to the fluid transfer device **11A** and the components therein, there is a proximal end and a distal end. The proximal end is that end closest to the fluid vessel into or from which fluid is transferred. In Fig. 13, the proximal end of the device is the end of the device where the plug **59A** is found. The distal end of the device is that end furthest away from the fluid vessel into or from which fluid is transferred and in Fig. 13, the distal end is the end where tab **107A** is found.

With continued reference to Fig. 13, the longitudinally displaceable shaft **81A** (referred to herein occasionally as just "shaft") has a proximal and distal end that corresponds with the proximal and distal ends of the passage. In operation, the shaft travels between a first position and a second position. As shown in Fig. 13, the shaft is in its first position. The shaft extends through and is sealingly attached to a diaphragm **29A.** The shaft is attached to tab assembly **113A** on the distal end of the device. A plug **59A** is attached to the proximal end of the shaft. The plug **59A** is secured to the shaft using supports **173A.** The plug may be constructed from silicone or, preferably, from platinum-cured silicone. The plug may also be constructed from a solvent resistant fluoroelastomer, such as a perfluoropolyether elastomer. However, it is understood that plug **59A** may be constructed of any suitable material. In Fig. 13, the plug **59A** is located outside the proximal end of the passage prior to displacement of the shaft from its first position. However, the plug **59A** may also be located at least partially within the inside the passage prior to displacement of the shaft from its first position. The plug may be shaped and located to effectively seal the proximal end of the passage when the shaft is in its first position.

Fluid transfer opening **167A** is located in the passage between the proximal opening of the passage and the diaphragm **29A** and provides a pathway for fluids to travel upon actuation of the device. A fluid transfer conduit **169A** is sealingly attached to the fluid transfer opening and provides a further pathway for fluid to travel. The combination of the fluid transfer opening **167A** and fluid transfer conduit **169A** allows for fluid to move into the fluid transfer opening **167A** and to the flexible tubing **13A** and then on to sample containers **19A** (not shown in Fig. 13). Alternatively, when fluid or media is transferred into a fluid vessel, the fluid pathway is essentially reversed. Flexible tubing **13A** may be securely attached to the fluid transfer conduit **169A** using, for example, a crimped collar **21A.** The space along the passage between the plug **59A** and diaphragm **29A** creates a chamber **161A.** This chamber remains substantially aseptic throughout operation and acts to keep the fluid transfer within a closed system from the point the sampled fluid leaves the fluid vessel to its arrival in a sample container.

With continued reference to Fig. 13, the shaft is disposed within a body that is constructed from an upper body portion **151A** and a lower body portion **83A** that, when joined together, form a unitary body. It should be understood that the body may be formed as a single structure or from two or more portions. The purpose of the body is to enclose and create a passageway in which the components of the fluid transfer device are located and thus the construction of the body may vary to accommodate the number of fluid transfer devices joined in an assembly. As shown, the body has a substantially cylindrical outer portion. Such shape provides for a compact organization of fluid transfer devices, is easily handled, and is similar in shape to the tank mount. Preferably, the body is constructed from high temperature glass reinforced polyester. Alternatively, the body may be constructed of polyvinylidene fluoride, polycarbonate (which provides high temperature resistance), polyether ether ketone (PEEK), or Ultem® (polyetherimide). However, it should be understood that the body may be constructed of any suitable material and its construction is not limited to the materials listed herein. Preferably the body, and in particular the lower body portion **83A**, contains an alignment aperture **171A** that guides the shaft while moving between its first and second positions.

Also depicted in Fig. 13 is tank mount **121A.** The fluid sample device **11A** or fluid sample device assembly **10A**, as shown in Fig. 11, is joined to a tank mount that allows the fluid sample device or assembly to be joined to any number of fluid vessels that are used in industrial settings. The tank mount is preferably constructed of 316L stainless steel. However, it should be understood that the tank mount may be constructed of any suitable material. The tank mount may also include a groove **139A** in which a seal is located to assist with a substantially aseptic connection of the fluid transfer device to a fluid vessel.

In operation the shaft **81A** moves between two positions. In its first position, the shaft is displaced toward the distal end of the passage, as shown in Fig. 13. The shaft **81A** may be biased in a first position that displaces the shaft toward the distal end of the passage. In other words, the shaft **81A** is forced in the distal direction and the plug **59A** seals the proximal end of the passage. The bias on the shaft **81A** may be created by, for example, the placement of a spring **79A**, such as shown in Fig. 13. While the shaft is in this first position the plug **59A** seals the passage through the body of the device at its proximal end. The diaphragm **29A** seals the passage at a location distal from the plug **59A** and thus creates the substantially aseptic chamber **161A.**

In its second position, as shown in Fig. 15, the shaft is displaced in a proximal direction toward the proximal end of the passage, for example, by a user depressing the tab and pushing the shaft proximally into a fluid vessel. Once displaced, the plug **59A** no longer seals the passage and a fluid pathway is created through the proximal end of the passage, into the chamber **161A**, through the fluid transfer opening **167A** and out the fluid transfer conduit **169A.** The fluid then travels through the flexible tubing **13A** and into a fluid sample container. The shaft is maintained in this second position until a sufficient sample of fluid is collected. Alternatively, fluid or media may be added to a fluid vessel by forcing the fluid or media to be added along this fluid pathway and into the fluid vessel.

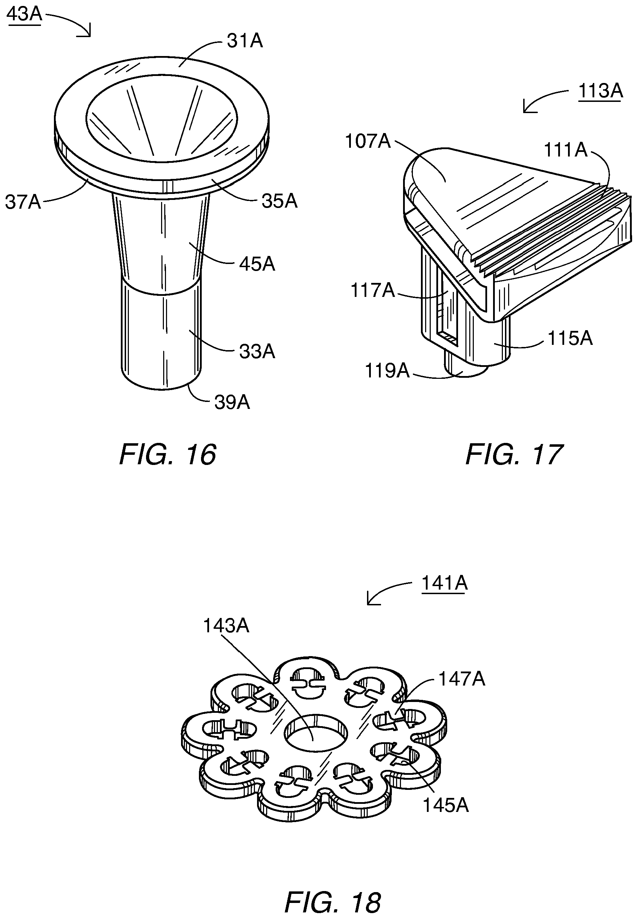

When the shaft is in its second position the diaphragm **29A** stretches while maintaining a seal around the shaft. Fig. 16 illustrates the diaphragm as it appears when stretched along its axis. The stretched diaphragm **43A** is made of flexible material and is capable of stretching in at least the direction of its axis. In this illustration, the stretched diaphragm **43A** has a top face **31A**, a central body **33A**, a side **35A**, an annular bead **37A**, and a central opening **39A.** The central opening **39A** is substantially cylindrical to receive the shaft **81A.** The body receives the annular bead **37A** in a seat **85A** as shown in Fig. 13. As used in the fluid transfer device disclosed herein, a shaft (see Figs. 13, 15, 19 and 20) extends through the central opening **39A** and is secured to the diaphragm **29A** by molding, for example, the diaphragm **29A** around the shaft when the diaphragm is produced. However, the diaphragm **29A** may be secured to a shaft through other means such as with adhesives or sealants. It is further understood that the central opening **39A** may have various shapes.

As shown in Fig. 17, a tab assembly **113A** comprises a tab **107A**, ridges **111A** to facilitate operation, a tab guide **115A** to guide the tab during use, and an axial channel **117A** in the tab guide **115A.** The shaft **81A** may be connected to the tab at the opening **119A.** Furthermore, a spring, such as that depicted in Fig. 13, may be fitted around the extension in which opening **119A** is located. Such arrangement is depicted in detail in Fig. 13.

Fig. 18 illustrates a retaining cap **141A**, a central opening **143A**, and openings **145A** through which tab guides, such as those depicted in Fig. 17, pass, and detents **147A.** In an assembled fluid transfer device, the retaining cap **141A** is located at the distal end of the upper portion of the body as shown in Fig. 13. The retaining cap **141A** may be constructed of a flexible material, such as polycarbonate or polyolefin. Preferably, the retaining cap is constructed of polycarbonate. However, the retaining cap may be constructed from any suitable material. The detents **147A** travel in the axial channel **117A** of the tab guide **115A.** Further, the bottom surface of tab **107A**, that is, the side opposite the ridges **111A**, contacts the top of the retaining cap **141A**, thereby limiting the axial travel of tab assembly **113A.** After a sample is taken, and when the tab **107A** is retracted in a distal direction, the extended detents **147A** keep the tab assembly **113A** from coming out of the retaining cap **141A** and potentially tearing the diaphragm and breaking the substantially aseptic state of the fluid transfer device and, in particular, the substantially aseptic chamber **161A.**

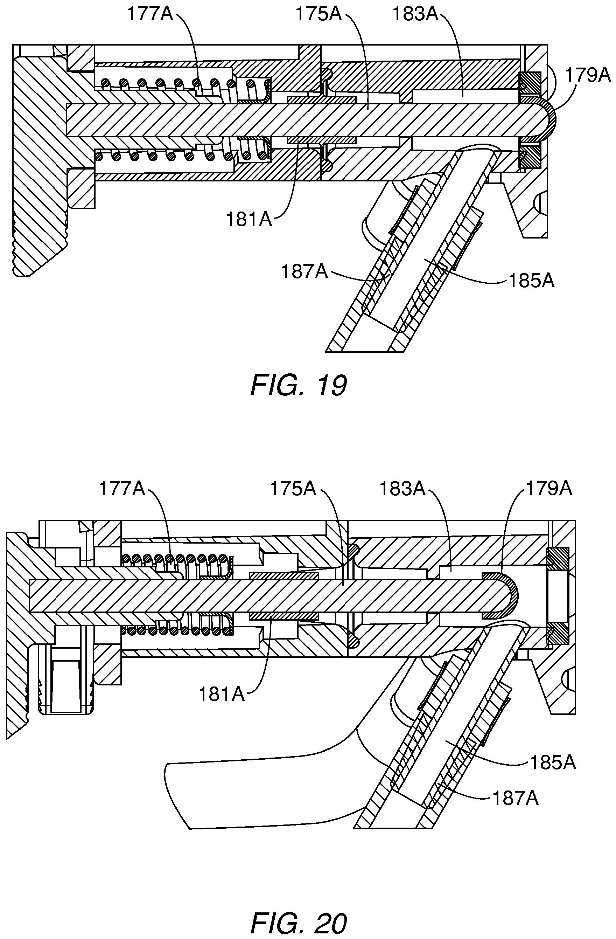

In another example of a fluid transfer device not covered by the present invention, and as disclosed in Fig. 19, a longitudinally displaceable shaft **175A** is disposed in and extends along a passage extending through the body of a fluid transfer device. The shaft **175A** moves between two positions wherein in its first position the shaft is displaced toward the proximal end of the passage. The shaft **175A** may be biased in a first position that displaces the shaft toward the proximal end of the passage. The bias may be created, for example, by the placement of a spring **177A**, such as shown in Fig. 19. While the shaft is in this first position a plug **179A** seals the passage through the body of the device at its proximal end. Plug **179A** is attached to the proximal end of the shaft **175A.** A diaphragm **181A** seals the passage at a location distal from the plug. The portion of the passage between the plug **179A** and diaphragm **181A** is a chamber **183A** that is substantially aseptic. A fluid transfer opening **185A** is located in the passage between the proximal opening of the passage and the diaphragm **29A** and provides a pathway for fluids to travel upon actuation of the device. A fluid transfer conduit **187A** is sealingly attached to the fluid transfer opening **185A** and provides a further pathway for fluid to travel. The combination of the fluid transfer opening **185A** and the fluid transfer conduit **187A** allows for fluid to move into or out of the fluid transfer opening **185A** and to the flexible tubing, which may be secured to the fluid transfer conduit 187A as described above.

In its second position, as shown in Fig. 20, the shaft is displaced toward the distal end of the passage, for example, by the user pulling the tab and thus pulling the shaft **175A** and plug **179A** distally away from a fluid vessel. Once displaced, the shaft **175A** and plug **179A** no longer create a seal at the proximal end of the passage and fluid now enters the passage up to the location of the diaphragm **181A** which maintains a seal around the distally displaced shaft. A fluid flow path is now created between the open end of the passage and the fluid transfer opening **185A** and through the fluid transfer conduit **187A.** The fluid then travels through the flexible tubing and into a fluid sample container. The shaft is left in this second position until a sufficient sample of fluid is collected. Alternatively, the device in this example may be used to transfer fluid or media into a fluid vessel as discussed in greater detail above. When the shaft is in its second position the diaphragm **181A** stretches while maintaining a seal around the shaft. Fig. 16 illustrates the diaphragm as it appears when stretched along its axis.

The fluid transfer devices disclosed herein may be sold pre-sterilized. Preferably, the entire passageway that fluid will travel is substantially aseptic until use. For example, and referring to Figs. 3 and 13, the tank mounts **121** or **121A,** plugs **59** or **59A**, shafts **81** or **81A**, passages **161** or **161A**, diaphragms **29** or **29A**, and flexible tubing **13** or **13A** are all substantially aseptic. By rendering and maintaining the pathway that fluid travels substantially aseptic, the fluid may be transferred from one vessel to another without risk of contamination to the fluid. Further, the unique aspect of chambers **161** and **161A** prevents the contamination of the environment around the fluid transfer device and maintains a completely closed fluid pathway before, during, and after actuation of the devices.

Also provided is a kit containing fluid transfer device assemblies **10** or **10A** as described herein, that is, comprising one or more bodies joined together, one or more lengths of flexible tubing **13** or **13A** as described herein, a plurality of sample containers **19** or **19A** as described herein, and tank mounts **121** or **121A** as described herein.

The fluid transfer device assembly **10** or **10A** may be assembled and then the entire devices or components thereof may be rendered substantially aseptic by, for example, gamma radiation. Alternatively, the entire devices or components thereof may be rendered substantially aseptic by exposure to steam above 121 °C for a period of time long enough to eliminate microorganisms. The entire devices or components thereof may also be rendered aseptic by chemical treatment, such as with ethylene oxide (ETO). Once rendered substantially aseptic, the devices may be appropriately packaged and stored to maintain the substantially aseptic state until ready for use.

A further advantage to the fluid transfer devices disclosed herein is the absence of pumps or other mechanical means to transfer fluids. The devices disclosed herein transfers fluid using gravity and the existing pressure inside a fluid vessel. The absence of pumps or other mechanical means fewer surfaces to render substantially aseptic and that particulates in the fluid are not damaged by the forces often exerted by mechanical pumps.

All dimensional information presented herein and included in the drawings is intended to be illustrative and not intended to limit the scope of the invention.

The foregoing descriptions of fluid transfer devices illustrate and describe various examples considered to represent best modes of carrying out the invention. As various changes can be made in the above examples without departing from the scope of the fluid transfer device claimed herein, it is intended that all matter contained in the above description or shown in the accompanying drawings shall be interpreted as illustrative and not limiting.

## Claims

1. A fluid transfer device (11) comprising:
a body (83, 151);
an elongate passage extending through the body (83, 151) and having a proximal end and a distal end;
a longitudinally displaceable hollow shaft (81) disposed in and extending along the passage, the shaft (81) having a proximal end and distal end and having in use a first position displaced toward the distal end of the passage and a second position displaced toward the proximal end of the passage, wherein the shaft (81) is axially biased longitudinally away from the proximal end of the passage into the first position;
a plug (59) attached to the proximal end of the shaft (81) and sealing the passage at the proximal end thereof when the shaft (81) is in the first position;
a diaphragm (29) sealing the passage at a location intermediate the plug (59) and the distal end of the passage;
the shaft (81) extending through and being sealingly secured to the diaphragm (29);
a fluid transfer opening (167) in the shaft (81) between the plug (59) and the diaphragm (29);
wherein longitudinal displacement of the shaft (81) towards its second position moves the plug (59) to open the passage, the diaphragm (29) stretching to accommodate the displacement of and maintain a seal about the shaft (81), a fluid flow path being established through the fluid transfer opening (167) in the shaft (81) and through its hollow interior.

2. The device of claim 1, wherein the portion of the passage between the plug (59) and diaphragm (29) is aseptic.

3. The device (11) of claim 1, wherein the plug (59) is disposed at least partially outside the proximal end of the passage prior to displacement of the shaft (81) from its first position.

4. The device of claim 1, comprising:
a plurality of bodies (10) joined together, each body (10) comprising:
an elongate passage extending through the body (10) and having a proximal end and a distal end;
a longitudinally displaceable hollow shaft (81) disposed in and extending along the passage, the shaft (81) having a proximal end and distal end and having in use a first position displaced toward the distal end of the passage and a second position displaced toward the proximal end of the passage, wherein the shaft (81) is axially biased longitudinally toward the distal end of the passage into the first position;
a plug (59) attached to the proximal end of the shaft and sealing the passage at the proximal end thereof when the shaft is in its first position;
a diaphragm (29) sealing the passage at a location intermediate the plug and the distal end of the passage;
the shaft (81) extending through and being sealingly secured to the diaphragm (29);
a fluid transfer opening in the shaft between the plug (59) and the diaphragm (29);
wherein longitudinal displacement of the shaft (81) towards its second position moves the plug (59) to open the passage, the diaphragm (29) stretching to accommodate the displacement of and maintain a seal about the shaft (81), a fluid flow path being established through the fluid transfer opening in the shaft and through its hollow interior.

5. A kit for transferring fluid comprising:
the fluid transfer device of claim 1;
a tank mount (121);
one or more lengths of flexible tubing; and
a plurality of sample containers.

## Patentansprüche

1. Fluidtransfervorrichtung (11), die Folgendes umfasst:
einen Körper (83, 151);
einen länglichen Kanal, der sich durch den Körper (83, 151) erstreckt und ein proximales Ende und ein distales Ende aufweist;
eine in Längsrichtung versetzbare hohle Welle (81), die im Kanal angeordnet ist und sich entlang desselben erstreckt, wobei die Welle (81) ein proximales Ende und ein distales Ende aufweist und im Gebrauch eine erste Position, die zum distalen Ende des Kanals hin versetzt ist, und eine zweite Position, die zum proximalen Ende des Kanals hin versetzt ist, aufweist, wobei die Welle (81) in Längsrichtung vom proximalen Ende des Kanals weg in die erste Position axial vorgespannt ist;
einen Stopfen (59), der am proximalen Ende der Welle (81) befestigt ist und den Kanal am proximalen Ende davon abdichtet, wenn sich die Welle (81) in der ersten Position befindet;
eine Membran (29), die den Kanal an einer Stelle zwischen dem Stopfen (59) und dem distalen Ende des Kanals abdichtet;
die Welle (81), die sich durch die Membran (29) erstreckt und abdichtend an dieser gesichert ist;
eine Fluidtransferöffnung (167) in der Welle (81) zwischen dem Stopfen (59) und der Membran (29);
wobei der Längsversatz der Welle (81) zu ihrer zweiten Position den Stopfen (59) bewegt, um den Kanal zu öffnen, wobei sich die Membran (29) dehnt, um den Versatz der Welle (81) aufzunehmen und eine Dichtung um dieselbe aufrechtzuerhalten, wobei ein Fluidströmungspfad durch die Fluidtransferöffnung (167) in der Welle (81) und durch ihren hohlen Innenraum aufgebaut wird.

2. Vorrichtung nach Anspruch 1, wobei der Abschnitt des Kanals zwischen dem Stopfen (59) und der Membran (29) aseptisch ist.

3. Vorrichtung (11) nach Anspruch 1, wobei der Stopfen (59) vor dem Versatz der Welle (81) aus ihrer ersten Position mindestens teilweise außerhalb des proximalen Endes des Kanals angeordnet ist.

4. Vorrichtung nach Anspruch 1, die Folgendes umfasst:
eine Vielzahl von Körpern (10), die verbunden sind, wobei jeder Körper (10) Folgendes umfasst:
einen länglichen Kanal, der sich durch den Körper (10) erstreckt und ein proximales Ende und ein distales Ende aufweist;
eine in Längsrichtung versetzbare hohle Welle (81), die im Kanal angeordnet ist und sich entlang desselben erstreckt, wobei die Welle (81) ein proximales Ende und ein distales Ende aufweist und im Gebrauch eine erste Position, die zum distalen Ende des Kanals hin versetzt ist, und eine zweite Position, die zum proximalen Ende des Kanals hin versetzt ist, aufweist, wobei die Welle (81) in Längsrichtung zum distalen Ende des Kanals in die erste Position axial vorgespannt ist;
einen Stopfen (59), der am proximalen Ende der Welle befestigt ist und den Kanal am proximalen Ende davon abdichtet, wenn sich die Welle in ihrer ersten Position befindet;
eine Membran (29), die den Kanal an einer Stelle zwischen dem Stopfen und dem distalen Ende des Kanals abdichtet;
die Welle (81), die sich durch die Membran (29) erstreckt und abdichtend an dieser gesichert ist;
eine Fluidtransferöffnung in der Welle zwischen dem Stopfen (59) und der Membran (29);
wobei der Längsversatz der Welle (81) zu ihrer zweiten Position den Stopfen (59) bewegt, um den Kanal zu öffnen, wobei sich die Membran (29) dehnt, um den Versatz der Welle (81) aufzunehmen und eine Dichtung um dieselbe aufrechtzuerhalten, wobei ein Fluidströmungspfad durch die Fluidtransferöffnung in der Welle und durch ihren hohlen Innenraum aufgebaut wird.

5. Kit zum Transferieren eines Fluids, das Folgendes umfasst:
eine Fluidtransfervorrichtung nach Anspruch 1;
eine Tankhalterung (121);
eine oder mehrere Längen eines flexiblen Schlauchs; und
eine Vielzahl von Probenbehältern.

## Revendications

1. Dispositif de transfert de fluide (11) comprenant :
un corps (83, 151) ;
un passage allongé s'étendant à travers le corps (83, 151) et ayant une extrémité proximale et une extrémité distale ;
un arbre creux longitudinalement déplaçable (81) disposé dans et s'étendant le long du passage, l'arbre (81) ayant une extrémité proximale et une extrémité distale et ayant, à l'usage, une première position déplacée vers l'extrémité distale du passage et une seconde position déplacée vers l'extrémité proximale du passage, dans lequel l'arbre (81) est sollicité axialement, longitudinalement à distance de l'extrémité proximale du passage dans la première position ;
un bouchon (59) fixé sur l'extrémité proximale de l'arbre (81) et scellant le passage au niveau de son extrémité proximale lorsque l'arbre (81) est dans la première position ;
un diaphragme (29) scellant le passage à un emplacement entre le bouchon (59) et l'extrémité distale du passage ;
l'arbre (81) s'étendant à travers et étant fixé, de manière étanche, sur le diaphragme (29) ;
une ouverture de transfert de fluide (167) dans l'arbre (81) entre le bouchon (59) et le diaphragme (29) ;
dans lequel le déplacement longitudinal de l'arbre (81) vers sa seconde position déplace le bouchon (59) pour ouvrir le passage, le diaphragme (29) s'étirant pour accepter le déplacement de et maintenir un joint d'étanchéité autour de l'arbre (81), une trajectoire d'écoulement de fluide étant établie à travers l'ouverture de transfert de fluide (167) dans l'arbre (81) et à travers son intérieur creux.

2. Dispositif selon la revendication 1, dans lequel la partie du passage entre le bouchon (59) et le diaphragme (29) est aseptique.

3. Dispositif (11) selon la revendication 1, dans lequel le bouchon (59) est disposé au moins partiellement à l'extérieur de l'extrémité proximale du passage avant le déplacement de l'arbre (81) de sa première position.

4. Dispositif selon la revendication 1, comprenant :
une pluralité de corps (10) assemblés ensemble, chaque corps (10) comprenant :
un passage allongé s'étendant à travers le corps (10) et ayant une extrémité proximale et une extrémité distale ;
un arbre creux longitudinalement déplaçable (81) disposé dans et s'étendant le long du passage, l'arbre (81) ayant une extrémité proximale et une extrémité distale et ayant, à l'usage, une première position déplacée vers l'extrémité distale du passage et une seconde position déplacée vers l'extrémité proximale du passage, dans lequel l'arbre (81) est axialement sollicité longitudinalement vers l'extrémité distale du passage dans la première position ;
un bouchon (59) fixé sur l'extrémité proximale de l'arbre et scellant le passage au niveau de son extrémité proximale lorsque l'arbre est dans sa première position ;
un diaphragme (29) scellant le passage à un emplacement entre le bouchon et l'extrémité distale du passage ;
l'arbre (81) s'étendant à travers et étant fixé de manière étanche sur le diaphragme (29) ;
une ouverture de transfert de fluide dans l'arbre entre le bouchon (59) et le diaphragme (29) ;
dans lequel le déplacement longitudinal de l'arbre (81) vers la seconde position déplace le bouchon (59) pour ouvrir le passage, le diaphragme (29) s'étirant pour accepter le déplacement de et le maintien d'un joint d'étanchéité autour de l'arbre (81), une trajectoire d'écoulement de fluide étant établie à travers l'ouverture de transfert de fluide dans l'arbre et à travers son intérieur creux.

5. Kit pour transférer du fluide comprenant :
un dispositif de transfert de fluide selon la revendication 1 ;
un support de réservoir (121) ;
une ou plusieurs longueurs de tube flexible ; et
une pluralité de récipients d'échantillon.
